# EUROPEAN PATENT APPLICATION

(11) **EP 3 888 636 A2**
(43) Date of publication of application: **06.10.2021**
(21) Application number: 19891363.4
(22) Date of filing: 26.11.2019
(51) Int. Cl.: A61K 9/14, A61K 31/445, A61K 9/70, A61P 25/28, A61K 9/08, A61K 47/08, A61K 47/12

(54) **DONEPEZIL EUTECTIC MIXTURE AND USE THEREOF**

(30) Priority: 26.11.2018 KR 20180147658; 16.09.2019 KR 20190113596
(71) Applicant: Ebabio Inc., Incheon 21984 (KR)
(72) Inventor: HWANG, Sung Joo, Seoul 05794 (KR); HYUN, Sang Min, Seogwipo-si, Jeju-do 63613 (KR); ABUZAR, Sharif Md, Incheon 21442 (KR); LEE, Soo Hun, Ulsan 44036 (KR); LEE, Eun Seok, Suwon-si Gyeonggi-do 16278 (KR); SUN, Bo Kyung, Seoul 06800 (KR)
(74) Representative: Treeby, Philip David William
(86) International application number: PCT/KR2019/016349
(87) International publication number: WO 2020/111719

(57) **Abstract**

The present invention relates to a donepezil eutectic mixture and a use thereof. A eutectic mixture of the present invention allows increased solubility and skin permeability of donepezil, and a pharmaceutical composition including the same does not generate donepezil crystals and maintains amorphous form even when stored under various conditions, thereby being applicable to various formulations while also having excellent stability and efficacy.

## Description

### [Technical Field]

The present invention relates to a donepezil eutectic mixture and a use thereof.

### [Background Art]

Alzheimer's disease is the most common degenerative brain disorder that causes dementia and is accompanied by symptoms such as personality change, nervous behavior, and depression as well as cognitive decline, and neurological disorders or physical complications appear at the end. Donepezil is used as a drug to treat Alzheimer's disease and is commercially available in tablet formulations containing the form of donepezil hydrochloride.

Donepezil is an acetylcholinesterase (AChE) inhibitor and is used for the treatment of dementia, such as Alzheimer's disease with a mild to severe degree or higher. In Alzheimer's disease, in association with which cholinergic nervous system disorders in the brain have been reported, AChE inhibitors, such as donepezil, increase acetylcholine in the brain to activate the cholinergic nerves in the brain. Donepezil, which is currently commonly used, is in the form of a tablet, and is prescribed to patients with Alzheimer's disease in the form of an oral preparation.

However, in general, the acetylcholinesterase inhibitor as an oral preparation has serious side effects, and in particular, is reported to cause problems such as hepatic dysfunction or digestive disorders. The cause of the side effects is that an oral preparation cannot generally avoid an effect of the primary passage into the liver, and as a result, the oral preparation may easily affect liver functions. Also, the oral preparation presents at a high concentration in the digestive tract, and thus, side effects are likely to occur in the digestive tract. In addition, with respect to a change in the blood drug concentration after administration of an oral preparation, the ratio of the maximum blood concentration reached after administration to the blood concentration 24 hours later (i.e., at the time of subsequent administration) is high, and thus, it is not easy to maintain a therapeutic effect over a long period of time, while the blood concentration does not reach a concentration at which side effects occur.

In particular, most Alzheimer's patients refuse to take drugs or have difficulty in swallowing or chewing drugs due to dysphagia. However, commercially available donepezil hydrochloride has a characteristically pungent and bitter taste, which causes difficulties with normal drug consumption. Also, donepezil transdermal preparation uses a large amount of various permeation enhancers due to its low skin permeability, thereby causing skin irritation problems such as skin rash, or when the drug is stored in a hydrophobic matrix, solid crystals are generated, resulting in a decrease in adhesion, a non-uniform skin permeation rate, and storage problems.

Accordingly, there is a continuous demand for the development of preparations and formulations capable of increasing drug compliance in the application of donepezil drugs.

### [Prior Art Document]

### [Patent Documents]

(Patent Document 1) Korean Registration Patent No. 10-1770675
(Patent Document 2) Korean Registration Patent No. 10-1553207

### [Disclosure]

### [Technical Problem]

An objective of the present invention is to provide a donepezil eutectic mixture including donepezil and coformer.

Another objective of the present invention is to provide a pharmaceutical composition including the donepezil eutectic mixture.

Another objective of the present invention is to provide a method of manufacturing the donepezil eutectic mixture.

### [Technical Solution]

An aspect of the present invention for achieving the objective relates to a donepezil eutectic mixture including donepezil and coformer, wherein the coformer is dicarboxylic acid or vanilin.

Specifically, the dicarboxylic acid may be at least one selected from the group consisting of adipic acid, malic acid, malonic acid, succinic acid, tartaric acid, azelaic acid, glutaric acid, itaconic acid, maleic acid, phthalic acid, pimelic acid, sebacic acid, suberic acid, and α-ketoglutaric acid.

More specifically, the donepezil and the coformer may be included in a weight ratio of 100:1 to 1:4, and most specifically, may be included in a weight ratio of 10:1 to 1:2.

Also, specifically, the eutectic mixture may be in a fluidized state at -70 °C to 250 °C, most specifically, -30 °C to 150 °C.

In the present invention, donepezil or a salt thereof has a chemical name, that is, 2-[(1-benzyl-4-piperidinyl)methyl]-5,6-dimethoxyindan-1-one, and is an acetylcholinesterase inhibitor used as a drug for treatment of Alzheimer's and dementia.

In the present invention, the term "eutectic mixture" refers to a mixture that has a certain melting point and in which fine heterogeneous crystals are uniformly mixed. Each substance constituting the eutectic mixture may have differentiating physicochemical characteristics, for example, molecular weight, molecular structure, logP, melting point, or amount of heat absorption, and may increase the solubility or dissolution of poorly soluble drugs. The donepezil eutectic mixture of the present invention has a functional group structurally capable of hydrogen bonding or ionic bonding, and solubility and skin permeability of donepezil may be significantly improved by forming a eutectic mixture via interaction with coformers capable of these bonding.

In the present invention, the term "coformer" refers to a material that may form a donepezil eutectic mixture by acting, reacting, or bonding with donepezil which is a main component of a drug. The coformer may be vanilin or at least one dicarboxylic acid selected from the group consisting of adipic acid, malic acid, malonic acid, succinic acid, tartaric acid, azelaic acid, glutaric acid, itaconic acid, maleic acid, phthalic acid, pimelic acid, sebacic acid, suberic acid, and α-ketoglutaric acid, but is not limited thereto, and any material that may be conformed with donepezil to form a eutectic mixture may be included without limitation.

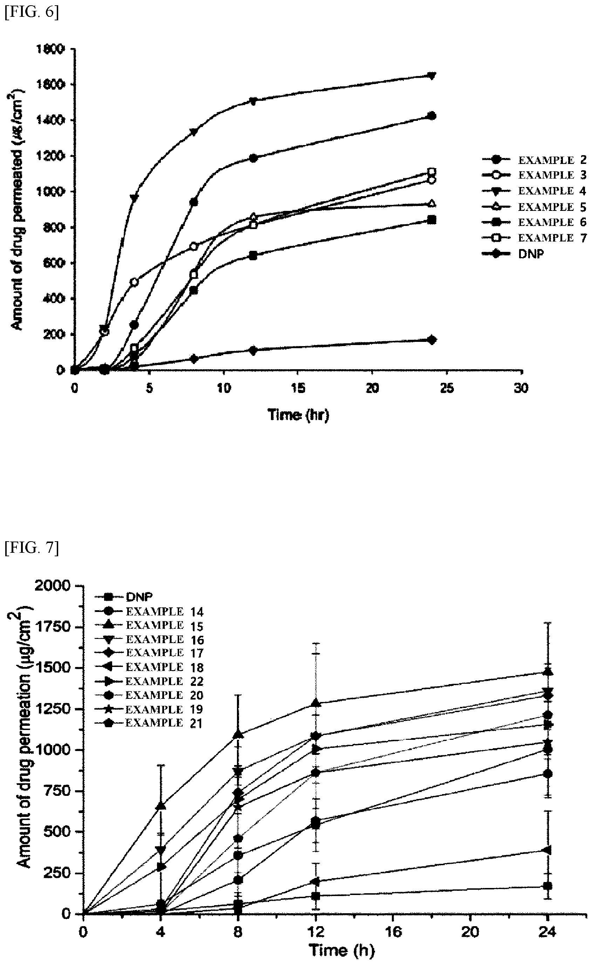

In an embodiment of the present invention, it was confirmed that solubility of all of eutectic mixtures of the present invention was significantly superior to that of commercially available donepezil raw material medicine (Table 5), it was also confirmed that skin permeability was significantly excellent (FIG. 6-8), and it was confirmed that the eutectic mixtures may be effectively applied not only in the form of oral administration preparations and injections, but also in a transdermal manner.

Another aspect of the present invention relates to a pharmaceutical composition for prevention or treatment of Alzheimer's or dementia, the pharmaceutical composition including the donepezil eutectic mixture.

Donepezil is conventionally utilized as a drug to treat Alzheimer's and dementia and to alleviate their symptoms, and the pharmaceutical composition including the donepezil eutectic mixture of the present invention may also be applied for prevention or treatment of Alzheimer's or dementia.

The pharmaceutical composition according to the present invention may be prepared in a pharmaceutical formulation using methods well known in the art so as to provide rapid, continuous, or delayed release of an active ingredient after administration to mammals. In preparation of the formulation, the pharmaceutical composition according to the present invention may further include a pharmaceutically acceptable carrier within a range that does not inhibit the activity of the donepezil eutectic mixture of the present invention.

The pharmaceutically acceptable carrier includes those commonly used, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil, but is not limited thereto. Also, the pharmaceutical composition of the present invention may include diluents or excipients such as filling agents, extenders, binders, wetting agents, disintegrating agents, and surfactants, and other pharmaceutically acceptable additives.

The pharmaceutical composition according to the present invention may be administered in a pharmaceutically effective amount. The term "pharmaceutically effective amount" refers to an amount sufficient to prevent or treat a disease at a reasonable benefit/risk ratio applicable to medical treatment. The effective dosage level may be variously selected by those skilled in the art according to factors such as formulation method, patient's condition and weight, patient's sex, age, degree of disease, drug type, route and duration of administration, excretion rate, and response sensitivity. The effective amount may vary depending on the route of treatment, the use of excipients, and the possibility of use with other medicaments, as is appreciated by those of skill in the art. However, for a desirable effect, in the case of oral administration, the composition of the present invention may be generally administered to an adult in amount of 0.0001 mg/kg to 100 mg/kg per day, preferably, 0.001 mg/kg to 100 mg/kg, based on 1 kg of body weight per day, but the dosage does not limit the scope of the present invention in any way.

The pharmaceutical composition of the present invention may be administered to mammals such as mice, livestock, and humans via various routes. Specifically, the pharmaceutical composition of the present invention may be administered orally or parenterally (for example, by application or intravenous, subcutaneous, or intraperitoneal injection), but oral administration is preferred. The pharmaceutical composition may be administered intravaginally in order to prevent or treat vaginitis. Solid preparations for oral administration may include powders, granules, tablets, capsules, soft capsules, pills, oral dissolving films, and the like. Liquid preparations for oral use may be suspensions, solvents, emulsions, syrups, aerosols, or the like, and may include various excipients, for example, humectants, sweeteners, fragrances, and preservatives, in addition water and liquid paraffin, which are commonly used simple diluents.

Preparations for parenteral administration may be used by being formulated in the form of sterile injection preparations and external preparations such as aqueous solutions, liquids, non-aqueous solutions, suspensions, emulsions, eye drops, ophthalmic ointment, syrups, suppositories, and aerosols, each sterilized according to conventional methods, and preferably, a pharmaceutical composition of cream, gel, patch, spray, ointment, plaster, lotion, liniment, ophthalmic ointment, eye drop, paste, or cataplasma may be prepared and used, but is limited thereto. Preparations for topical administration may be anhydrous or aqueous, depending on the clinical prescription. As the nonaqueous solvent and suspension, propylene glycol, polyethylene glycol, vegetable oil such as olive oil, injectable ester such as ethyl oleate, and the like may be used. As a base for suppositories, witepsol, macrogol, tween 61, cacao butter, laurin butter, glycerogelatin, and the like may be used.

Specifically, the pharmaceutical composition of the present invention may be a transdermal preparation.

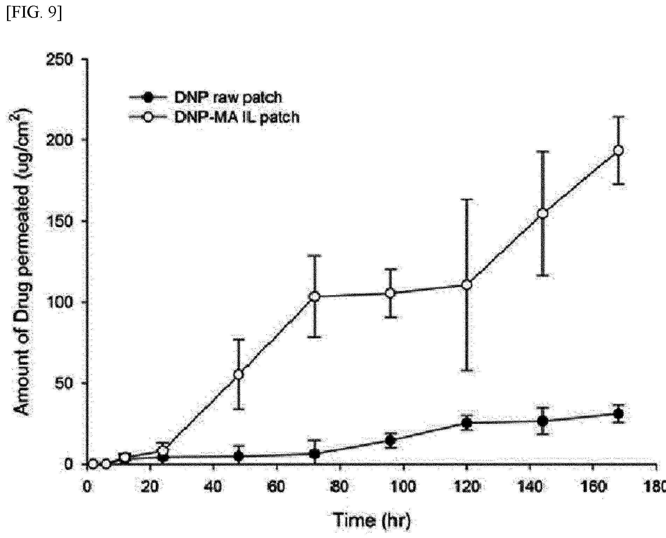

Also, the transdermal preparation may be in the form of a patch (reservior type) or a film (matrix type). In an embodiment of the present invention, it was confirmed that excellent skin permeability was shown even when a transdermal preparation is applied (FIG. 9), and it may be applied in a form that may be attached to a surface. The surface includes the skin of all parts of the body, the surface of the mouth, and the like, and is not limited to a specific part.

In the case of conventional patches, main ingredients (main drugs) are uniformly distributed in an additive of the patches to exhibit release characteristics of a certain reference, but as the main component is precipitated as crystals over time during distribution or storage, a desired medicinal efficacy is not exhibited and stability is poor. However, the eutectic mixture of the present invention maintains the amorphous form of the drug while crystals are not generated over time, maintains initial release characteristics and has excellent stability, by maintaining the amorphous form, and has excellent dissolution and excellent skin permeability.

According to another aspect of the present invention relates to a method of preventing or treating Alzheimer's or donepezil, the method including administering a pharmaceutical composition including the donepezil eutectic mixture to a subject. In the present invention, the terms "donepezil eutectic mixture" and the like are each the same as described above.

The subject refers to an animal, and typically, may be a mammal that may exhibit beneficial effects by treatment using the pharmaceutical composition of the present invention. Preferred examples of the subject may include primates such as humans. Also, such subjects may include all subjects that may have symptoms of Alzheimer's or dementia or are at risk of having the symptoms.

Another aspect of the present invention relates to a method of manufacturing a donepezil eutectic mixture, the method including mixing donepezil and coformer, wherein the coformer is dicarboxylic acid or vanilin. Specifically, the donepezil and the coformer may be included in a weight ratio of 100:1 to 1:4, and more specifically, may be included in a weight ratio of 10:1 to 1:2.

Also, specifically, the eutectic mixture may be in a fluidized state at -70 °C to 250 °C, most specifically, -30 °C to 150 °C.

In an embodiment of the present invention, a donepezil eutectic mixture is manufactured via the manufacturing method including mixing donepezil with coformer which is vanilin or at least one dicarboxylic acid selected from the group consisting of adipic acid, malic acid, malonic acid, succinic acid, tartaric acid, azelaic acid, glutaric acid, itaconic acid, maleic acid, phthalic acid, pimelic acid, sebacic acid, suberic acid, and α-ketoglutaric acid, and it was confirmed that the donepezil eutectic mixture showed excellent solubility and excellent skin permeability.

Accordingly, a donepezil eutectic mixture may be manufactured using the above-described manufacturing method and utilized for prevention and treatment of Alzheimer's or dementia.

### [Advantageous Effects]

A donepezil eutectic mixture of the present invention has significantly increased solubility and skin permeability compared to those of donepezil, and thus can increase drug compliance. Particularly, due to high skin permeability, a drug can be transdermally administered, and thus, improved therapeutic effect can be shown to Alzheimer's or dementia patients who have difficulty in drug consumption.

### [Description of Drawings]

FIG. 1 shows transdermal preparations prepared including Comparative Example 1 and Example 4, respectively (left: patch of Comparative Example 3, right: patch of Example 23).
FIG. 2 shows a result of confirming that Examples 2 to 7 of the present invention each exhibit fluidity at room temperature via differential scanning calorimetry analysis.
FIG. 3 shows a result of confirming that Example 14 to 22 of the present invention each exhibit fluidity at room temperature via differential scanning calorimetry analysis.
FIG. 4 shows a result of observing the state change of a eutectic mixture of Example 4 according to temperature.
FIG. 5 shows a result of observing the state change of a eutectic mixture of Example 15 according to temperature.
FIG. 6 shows a result of measuring skin permeability (i.e., skin permeability depending on types of coformer) of eutectic mixtures of Examples 2 to 7 and Comparative 1.
FIG. 7 shows a result of measuring skin permeability (i.e., skin permeability depending on types of coformer) of eutectic mixtures of Examples 14 to 22 and Comparative 1.
FIG. 8 shows a result of measuring skin permeability (i.e., skin permeability depending on a ratio of donepezil to malonic acid) of eutectic mixtures of Examples 8 to 13 and Comparative 1.
FIG. 9 shows a result of measuring skin permeability of transdermal preparations prepared including Comparative Example 1 and Example 4, respectively (DNP raw patch: patch of Comparative Example 3, DNP-MA IL patch: patch of Example 23).

### [Best Modes of the Invention]

Hereinafter, the present invention will be described in detail by examples. However, the following examples are merely illustrative of the present invention, and the present invention is not limited by the following examples.

### Example 1. Manufacture of eutectic mixture of donepezil-malonic acid (1:1)

100 mg of Donepezil (from Perrigo) and 100 mg malonic acid were added to a mortar, and then pulverized and mixed using a pestle for 30 minutes, to thereby prepare a composition.

### Example 2. Manufacture of eutectic mixture of donepezil-adipic acid (1:1)

100 mg of donepezil (from Perrigo) and 100 mg of adipic acid were added to an ethanol (EtOH) solvent (1 ml) and stirred at 25 °C for about 30 minutes to thereby prepare a mixed solution in which donepezil and adipic acid were dissolved. The solution was dried in an oven at 60 °C for 24 hours to evaporate the solvent, to thereby obtain a eutectic mixture of donepezil-adipic acid (1:1).

### Example 3. Manufacture of eutectic mixture of donepezil-malic acid (1:1)

100 mg of donepezil (from Perrigo) and 100 mg of malic acid were added to an ethanol (EtOH) solvent (1 ml) and stirred at 25 °C for about 30 minutes to thereby prepare a mixed solution in which donepezil and malic acid were dissolved. The solution was dried in an oven at 60 °C for 24 hours to evaporate the solvent, to thereby obtain a eutectic mixture of donepezil-malic acid (1:1).

### Example 4. Manufacture of eutectic mixture of donepezil-malonic acid (1:1)

100 mg of donepezil (from Perrigo) and 100 mg of malonic acid were added to an ethanol (EtOH) solvent (1 ml) and stirred at 25 °C for about 30 minutes to thereby prepare a mixed solution in which donepezil and malonic acid were dissolved. The solution was dried in an oven at 60 °C for 24 hours to evaporate the solvent, to thereby obtain a eutectic mixture of donepezil-malonic acid (1:1).

### Example 5. Manufacture of eutectic mixture of donepezil-succinic acid (1:1)

100 mg of donepezil (from Perrigo) and 100 mg of succinic acid were added to an ethanol (EtOH) solvent (1 ml) and stirred at 25 °C for about 30 minutes to thereby prepare a mixed solution in which donepezil and succinic acid were dissolved. The solution was dried in an oven at 60 °C for 24 hours to evaporate the solvent, to thereby obtain a eutectic mixture of donepezil-succinic acid (1:1).

### Example 6. Manufacture of eutectic mixture of donepezil-tartaric acid (1:1)

100 mg of donepezil (from Perrigo) and 100 mg of tartaric acid were added to an ethanol (EtOH) solvent (1 ml) and stirred at 25 °C for about 30 minutes to thereby prepare a mixed solution in which donepezil and tartaric acid were dissolved. The solution was dried in an oven at 60 °C for 24 hours to evaporate the solvent, to thereby obtain a eutectic mixture of donepezil-tartaric acid (1:1).

### Example 7. Manufacture of eutectic mixture of donepezil-vanilin (1:1)

100 mg of donepezil (from Perrigo) and 100 mg of vanilin were added to an ethanol (EtOH) solvent (1 ml) and stirred at 25 °C for about 30 minutes to thereby prepare a mixed solution in which donepezil and vanilin were dissolved. The solution was dried in an oven at 60 °C for 24 hours to evaporate the solvent, to thereby obtain a eutectic mixture of donepezil-vanilin (1:1).

### Examples 8 to 13. Manufacture of eutectic mixture according to donepezil-malonic acid ratio

Donepezil and malonic acid were added to an ethanol (EtOH) solvent (1 ml) at each ratio shown in the following table and stirred at 25 °C for about 30 minutes to thereby prepare a mixed solution in which donepezil and malonic acid were dissolved. The solution was dried in an oven at 60 °C for 24 hours to evaporate the solvent, to thereby obtain eutectic mixtures of Examples 8 to 13 in Table 1 below.

**[Table 1]**

| Category | Donepezil (mg) | Malonic acid (mg) |
|---|---|---|
| Example 8 | 100 | 109.6 |
| Example 9 | 100 | 63.9 |
| Example 10 | 100 | 41.0 |
| Example 11 | 100 | 27.4 |
| Example 12 | 100 | 8.3 |
| Example 13 | 100 | 11.7 |

### Example 14. Manufacture of eutectic mixture of donepezil (DNP)-azelaic acid

100 mg of donepezil (from Perrigo) and 100 mg of azelaic acid were added to an ethanol (EtOH) solvent (1 ml) and stirred at 25 °C for about 30 minutes to thereby prepare a mixed solution in which donepezil and azelaic acid were dissolved. The solution was dried in an oven at 60 °C for 24 hours to evaporate the solvent, to thereby obtain a eutectic mixture of donepezil-azelaic acid (1:1).

### Example 15. Manufacture of eutectic mixture of donepezil-glutaric acid

100 mg of donepezil (from Perrigo) and 100 mg of glutaric acid were added to an ethanol (EtOH) solvent (1 ml) and stirred at 25 °C for about 30 minutes to thereby prepare a mixed solution in which donepezil and glutaric acid were dissolved. The solution was dried in an oven at 60 °C for 24 hours to evaporate the solvent, to thereby obtain a eutectic mixture of donepezil-glutaric acid (1:1).

### Example 16. Manufacture of eutectic mixture of donepezil-itaconic acid

100 mg of donepezil (from Perrigo) and 100 mg of itaconic acid were added to an ethanol (EtOH) solvent (1 ml) and stirred at 25 °C for about 30 minutes to thereby prepare a mixed solution in which donepezil and itaconic acid were dissolved. The solution was dried in an oven at 60 °C for 24 hours to evaporate the solvent, to thereby obtain a eutectic mixture of donepezil-itaconic acid (1:1).

### Example 17. Manufacture of eutectic mixture of donepezil-maleic acid

100 mg of donepezil (from Perrigo) and 100 mg of maleic acid were added to an ethanol (EtOH) solvent (1 ml) and stirred at 25 °C for about 30 minutes to thereby prepare a mixed solution in which donepezil and maleic acid were dissolved. The solution was dried in an oven at 60 °C for 24 hours to evaporate the solvent, to thereby obtain a eutectic mixture of donepezil-maleic acid (1:1).

### Example 18. Manufacture of eutectic mixture of donepezil-phthalic acid

100 mg of donepezil (from Perrigo) and 100 mg of phthalic acid were added to an ethanol (EtOH) solvent (1 ml) and stirred at 25 °C for about 30 minutes to thereby prepare a mixed solution in which donepezil and phthalic acid were dissolved. The solution was dried in an oven at 60 °C for 24 hours to evaporate the solvent, to thereby obtain a eutectic mixture of donepezil-phthalic acid (1:1).

### Example 19. Manufacture of eutectic mixture of donepezil-pimelic acid

100 mg of donepezil (from Perrigo) and 100 mg of pimelic acid were added to an ethanol (EtOH) solvent (1 ml) and stirred at 25 °C for about 30 minutes to thereby prepare a mixed solution in which donepezil and pimelic acid were dissolved. The solution was dried in an oven at 60 °C for 24 hours to evaporate the solvent, to thereby obtain a eutectic mixture of donepezil-pimelic acid (1:1).

### Example 20. Manufacture of eutectic mixture of donepezil-sebacic acid

100 mg of donepezil (from Perrigo) and 100 mg of sebacic acid were added to an ethanol (EtOH) solvent (1 ml) and stirred at 25 °C for about 30 minutes to thereby prepare a mixed solution in which donepezil and sebacic acid were dissolved. The solution was dried in an oven at 60 °C for 24 hours to evaporate the solvent, to thereby obtain a eutectic mixture of donepezil-sebacic acid (1:1).

### Example 21. Manufacture of eutectic mixture of donepezil-suberic acid

100 mg of donepezil and 100 mg of suberic acid were added to an ethanol (EtOH) solvent (1 ml) and stirred at 25 °C for about 30 minutes to thereby prepare a mixed solution in which donepezil and suberic acid were dissolved. The solution was dried in an oven at 60 °C for 24 hours to evaporate the solvent, to thereby obtain a eutectic mixture of donepezil-suberic acid (1:1).

### Example 22. Manufacture of eutectic mixture of donepezil-α-ketoglutaric acid

100 mg of donepezil and 100 mg of α-ketoglutaric acid were added to an ethanol (EtOH) solvent (1 ml) and stirred at 25 °C for about 30 minutes to thereby prepare a mixed solution in which donepezil and α-ketoglutaric acid were dissolved. The solution was dried in an oven at 60 °C for 24 hours to evaporate the solvent, to thereby obtain a eutectic mixture of donepezil-α-ketoglutaric acid (1:1).

### Comparative Example 1. Donepezil raw material

A commercially available donepezil raw material was used as Comparative Example 1.

### Comparative Example 2. Donepezil hydrochloride

A commercially available donepezil hydrochloride raw material was used as Comparative Example 2.

### Preparation Example 1. Manufacture of transdermal preparation

Comparative Example 3 and Example 23 were prepared as follows.

Example 23 was prepared by homogeneously mixing components (donepezil, malonic acid, polyisobutylene, butylhydroxytoluen, mineral oil, and hydrocabon resin (Regalite R1100)) according to their amounts shown in Table 2 below, and a patch of Comparative Example 3 was prepared by homogeneously mixing donepezil raw material, polyisobutylene, butylhydroxytoluen, mineral oil, and hydrocabon resin (Regalite R1100) using a mixer. The obtained mixture was left unattended for 6 hours to remove air bubbles, applied to a release liner in an appropriate amount, and then dried at 50 °C for 2 hours and 70 °C for 30 minutes. Afterwards, a backing membrane was adhered thereto to thereby prepare a transdermal preparation.

**[Table 2]**

| Category | Component | Comparative Example 3 | | Example 23 | |
|---|---|---|---|---|---|
| | | % | mg | % | mg |
| Main component | Donepezil | 10.0 | 500 | 10.0 | 500 |
| Coformer | Malonic acid | - | - | 10.0 | 500 |
| Stabilizing agent | Butylhydroxytoluene (BHT) | 0.3 | 15 | 0.3 | 15 |
| Polymer | Oppanol™ B 100 (polyisobutylene, PIB) | 22.5 | 1125 | 20.85 | 1042.5 |
| | Oppanol™ B 15 | 22.5 | 1125 | 20.85 | 1042.5 |
| Excipient | Mineral oil | 17.8 | 890 | 14.4 | 720 |
| Tacktifier | Regalite R1100 (Hydrocarbon Resin) | 26.9 | 1345 | 23.6 | 1180 |
| Sum | | 100 | 5000 | 100 | 5000 |

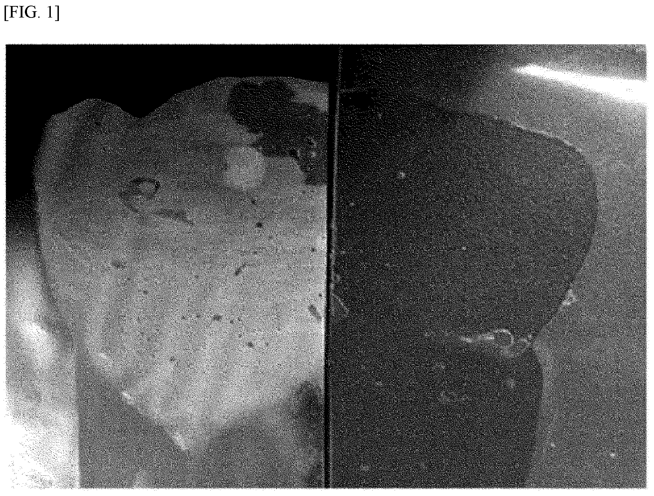

As shown in FIG. 1, the patch of the comparative example using the donepezil raw material was non-uniformly adhered to the surface due to a large amount of air bubbles while showing a turbid color and non-uniform drug distribution, whereas the patch of Example 23 prepared using the donepezil eutectic mixture showed a transparent color and uniform drug distribution, indicating that the patch was well adsorbed on the surface.

The above result indicates that, compared to conventional patches, the transdermal preparation including the donepezil eutectic mixture of the present invention maintains the amorphous form of the drug while crystals are not generated over time, has excellent stability and maintains initial release characteristics, by maintaining the amorphous form, and increases drug delivery effect by being well adhered to the surface.

### Experimental Example 1. Differential scanning calorimetry analysis

A differential scanning calorimeter (DSC auto Q2000, TA instrument, New castle, USA) was used to identify thermal behavior of samples. Specifically, 3 mg to 6 mg of all samples were accurately weighed and sealed in an aluminum pan. Measurements were made at a heating rate of 10 °C/min and a nitrogen flow rate of 40 ml/min, and a temperature range was appropriately selected from -70 °C to 250 °C for each material.

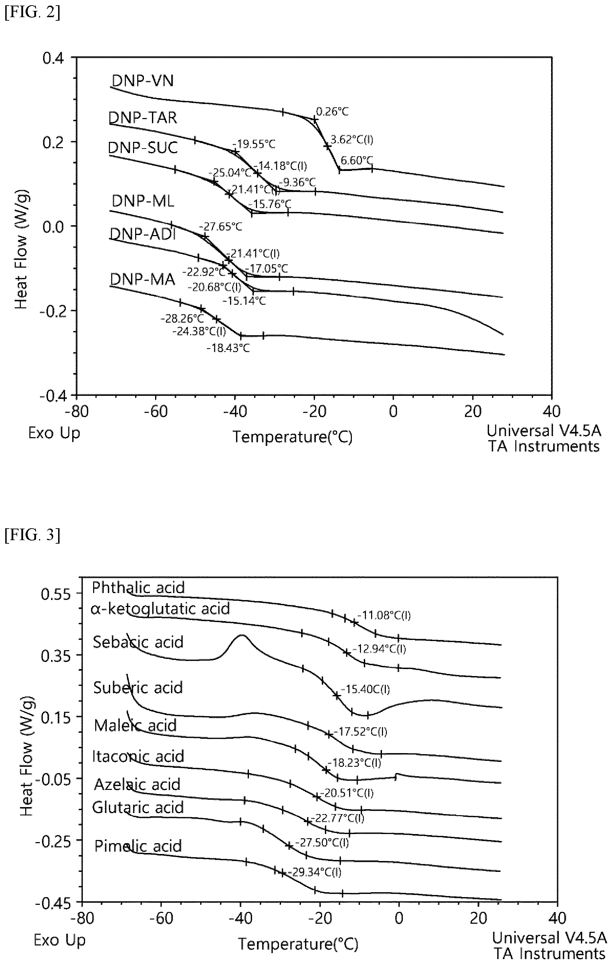

As a result of the measurements, it was conformed that Examples 2 to 7 and Examples 14 to 22 each had a glass transition temperature (Tg) below room temperature, which means that they have fluidity in a paste state or a liquid state (Table 3 and FIGS. 2 and 3).

**[Table 3]**

| | Sample name | Tg (°C) |
|---|---|---|
| Example 2 | DNP-ADI | -20.68 |
| Example 3 | DNP-MA | -24.38 |
| Example 4 | DNP-ML | -21.41 |
| Example 5 | DNP-SUC | -21.41 |
| Example 6 | DNP-TAR | -14.18 |
| Example 7 | DNP-VN | 3.62 |
| Example 14 | DNP-Azelaic acid | -22.77 |
| Example 15 | DNP-Glutaric acid | -27.50 |
| Example 16 | DNP-Itaconic acid | -20.51 |
| Example 17 | DNP-Maleic acid | -18.23 |
| Example 18 | DNP-Phthalic acid | -11.08 |
| Example 19 | DNP-Pimelic acid | -29.34 |
| Example 20 | DNP-Sebacic acid | -15.40 |
| Example 21 | DNP-Suberic acid | -17.52 |
| Example 22 | DNP-α-ketoglutaric acid | -12.94 |

### Experimental Example 2. Checking of state change according to temperature

In order to check the state change according to temperature, observation was made using a hot state microscopy capable of controlling temperature of samples. The model used was an Axio scope (A1,Carl zeiss, Oberkochen, Germany) and was performed at magnification of 200 times using a microscope camera (Axio cam MRc, Carl zeiss,Oberkochen Germany). When temperature control is required, the temperature was controlled at a heating rate of 5 °C/min.

Specifically, the state change of the eutectic mixture of Example 4 according to temperature was identified, and was measured at each temperature of 32 °C (a), 50 °C (b), 75 °C (c), 100 °C (d), 122.5 °C (e), and 140 °C (f).

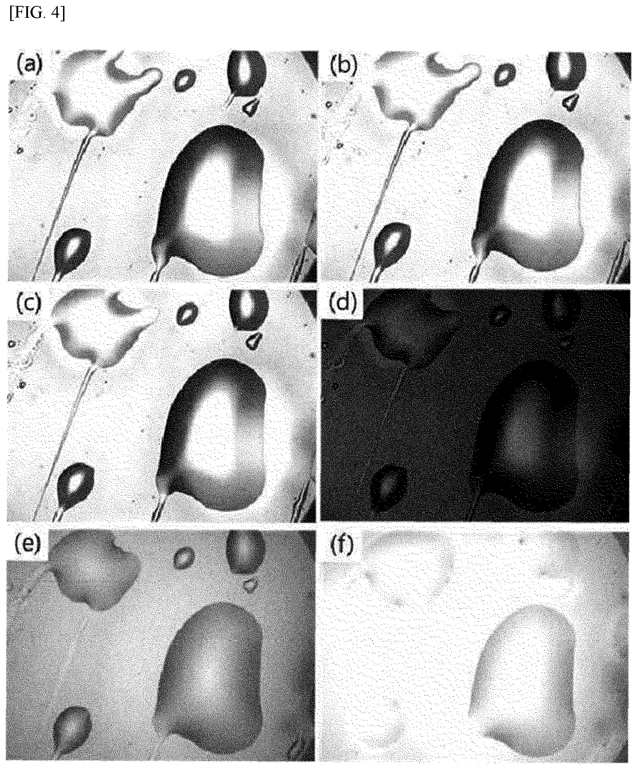

As a result, as shown in FIG. 4, it was observed that, since 32 °C (a), the eutectic mixture had already been in a liquid state or in a paste state having fluidity, which means that there was no significant difference from the state at 94 °C to 99 °C, which is the melting point of donepezil, and at 135 °C to 140 °C, which is the melting point of malonic acid.

This implies that the eutectic mixture of the present invention may prevent deformation of an active ingredient of drugs by obtaining a liquid property for improving drug absorption even when a high temperature is applied to donepezil or an organic solvent is not added.

Also, the state change of the eutectic mixture of Example 15 according to temperature was identified, and was observed at each temperature of 25 °C (a), 50 °C (b), 70 °C (c), 90 °C (d), 100 °C (e), and 110 °C (f).

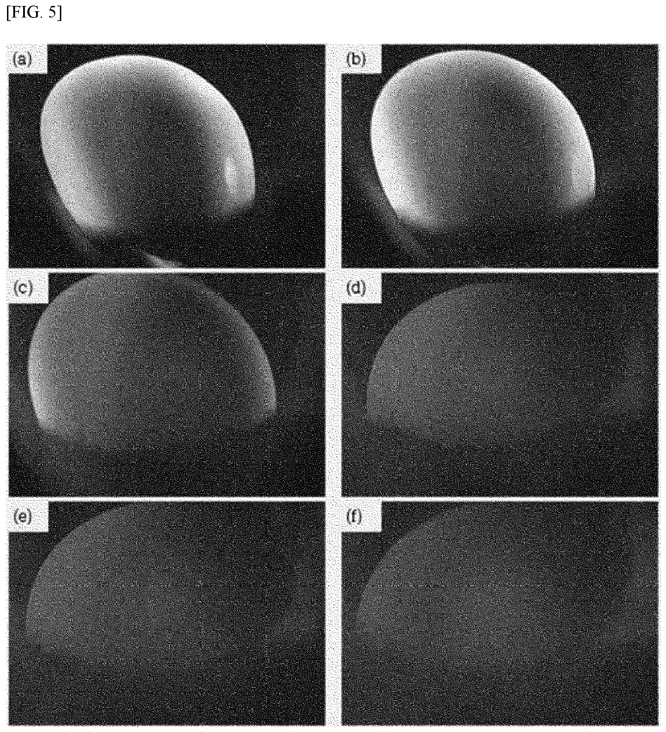

As a result, as shown in FIG. 5, it was confirmed that, since 25 °C (a), the eutectic mixture had already been in a liquid state or in a paste state having fluidity, which means that there was no significant difference from the state at 94 °C to 99 °C, which is the melting point of donepezil, and at 95 °C to 98 °C, which is the melting point of glutaric acid.

This means that the eutectic mixture including donepezil and coformer of the present invention may obtain a liquid property for improving drug absorption even when a high temperature is applied or an organic solvent is not added, and implies that crystal precipitation of a main component may be prevented when transdermal patches are prepared.

### Experimental Example 3. Solubility test

A solubility test was performed on the donepezil eutectic mixtures obtained in Examples 2 to 7 and Examples 14 to 22 and Comparative Example (donepezil raw material). Specifically, 100 mg of a sample corresponding to 50 mg of donepezil (50 mg of the donepezil raw material) was added to 0.5 ml of distilled water (at a concentration of 100 mg/mL) and stirred at a rate of 50 rpm at room temperature for 24 hours using a rotator (CRT-350, Lab companion). An additional 100 mg of a sample was added to a sample which was checked every 2 hours and was already transparently dissolved. In the case of melting greater than or equal to 1:1, the experiment was no longer conducted.

Solubility results were classified according to the solubility reference table of the following U.S. Pharmacopoeia.

**[Table 4]**

| Description | Solubility criteria (Parts of solvent required for one part of solute) |
|---|---|
| Very soluble | <1 |
| Freely soluble | 1-10 |
| Soluble | 10-30 |
| Sparingly soluble | 30-100 |
| Slightly soluble | 100-1000 |
| Very slightly soluble | 1000-10000 |
| Insoluble | >10000 |

The solubility test results according to the solubility reference table of the U.S. Pharmacopoeia are shown in Table 5 below.

**[Table 5]**

| Category | Sample name | Solubility at room temperature | |
|---|---|---|---|
| | | mg/mL | U.S. Pharmacopeia reference |
| Comparative Example 1 | DNP | 0.07 (74.55*µ*g/mL) | Insoluble |
| Comparative Example | DNP-HCl | 29.86 | Sparingly soluble |
| 2 | | | |
| Example 2 | DNP-ADI | 137.11 | Freely soluble |
| Example 3 | DNP-MA | Very soluble | |
| Example 4 | DNP-ML | Very soluble | |
| Example 5 | DNP-SUC | 276.81 | Freely soluble |
| Example 6 | DNP-TAR | Very soluble | |
| Example 7 | DNP-VN | 4.40 | Slightly soluble |
| Example 14 | DNP-Azelaic acid | 7.01 | Slightly soluble |
| Example 15 | DNP-Glutaric acid | 90.38 | Soluble |
| Example 16 | DNP-Itaconic acid | Very soluble | |
| Example 17 | DNP-Maleic acid | Very soluble | |
| Example 18 | DNP-Phthalic acid | 3.64 | Slightly soluble |
| Example 19 | DNP-Pimelic acid | Very soluble | |
| Example 20 | DNP-Sebacic acid | 9.93 | Slightly soluble |
| Example 21 | DNP-Suberic acid | 20.79 | Sparingly soluble |
| Example 22 | DNP-α-ketoglutaric acid | Very soluble | |

As shown in Table 5, the donepezil raw material (solubility: 74.55 *µ*g/mL) of Comparative Example 1 was found to be insoluble, whereas Examples 2 to 7 were found to have higher solubility than that of Comparative Example 1 by a factor of at least 60. Meanwhile, Examples 14 to 22 were found to have higher solubility than that of Comparative Example 1 by a factor of at least 50.

Particularly, Examples 2 to 6 were found to have superior solubility to that of donepezil hydrochloride of Comparative Example 2, which is a component of commercially available products. It was confirmed that Examples 3, 4, and 6 among them had solubility of at least 1 /mL and corresponded to "Very soluble" (Korean Pharmacopoeia) or "Very soluble" (USP).

### Experimental Example 4. Skin permeability measurement

### 4-1. Skin permeability measurement according to types of coformer

A skin permeability test was performed on the eutectic mixtures of Examples 2 to 7 and Comparative Example 1 (donepezil raw material) respectively synthesized by different types of coformer. Specifically, each eutectic mixture was applied to a release liner of 1x1 cm in an appropriate amount and then adhered to an artificial skin, which was then fixed to a Franz cell using a clamp. In the case of using a transdermal preparation, it was cut into a size of 1x1 cm and then used by being adhered to a skin. Isotonic phosphate buffer (pH 7.4) was added to a receptor, maintained at 32.5 °C, and then stirred at 300 rpm using a magnetic stirrer, 1 ml of a sample was collected at 0, 2, 4, 8, 12, and 24 hours (collection was made at 0, 2, 6, 12, 24, 48, 72, 96, 120, 144, and 168 hours in case of a test of skin permeability of a patch), and isotonic phosphate buffer was added again thereto. The obtained sample was quantified using high-speed liquid chromatography and an ultraviolet absorption spectrophotometer under the following analysis condition. The analysis condition is as follows.

### <Analysis condition>

Column: Phenomenex, 250*4.6*5, C18 column
Mobile phase: 0.1M PBS pH 2.7 solution : Acetonitrile : Methanol = 50 : 20 :30
Temperature: 25 °C
Amount of sample injected: 20 µL
UV absorption wavelength: 268 nm
Analysis time period: 7 minutes

As a result, as shown in FIG. 6, Comparative Example 1 showed little skin permeability over 24 hours, whereas the eutectic mixtures of Examples 2 to 7 showed higher skin permeability than that of Comparative Example 1 by a factor of at least 8 after 24 hours.

Particularly, Example 4 showed a skin permeability of 1,509 µg/cm2 in 12 hours, which is higher than that of Comparative Example 1 by a factor of about at least 14 in that same time.

Also, as shown in FIG. 7, Comparative Example 1 showed little skin permeability over 24 hours, whereas the eutectic mixtures of Examples 14 to 22 showed improved skin permeability compared to that of Comparative Example 1.

Particularly, Example 15 showed a skin permeability of 1,475 µg/cm2 for 24 hours, which is higher than that of Comparative Example 1 by a factor of about at least 8 in that same time.

The above results indicate that the eutectic mixture of the present invention exhibits excellent skin permeability, indicating that a transdermal drug administration, which is not applicable in Comparative Example 1, may be applied to the eutectic mixture of the present invention.

### 4-2. Measurement of skin permeability of eutectic mixtures with different ratios of donepezil to malonic acid

Skin permeability was measured with respect to the eutectic mixtures respectively prepared in Examples 8 to 13 and Comparative Example 1 (donepezil raw material). A specific skin permeability test was performed in the same manner as in the method described in 4-1.

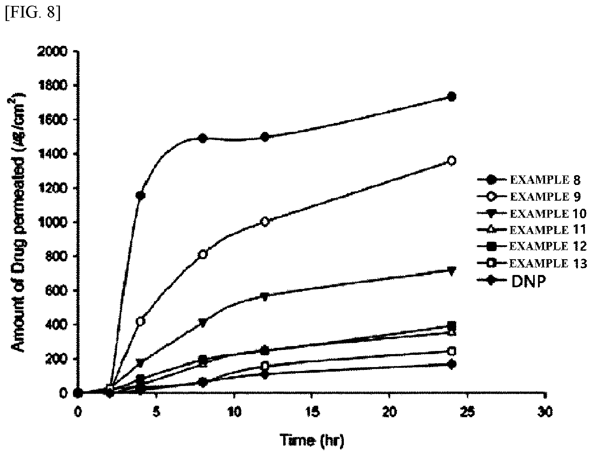

As a result, as shown in FIG. 8, it was confirmed that even when a proportion of malonic acid varied, all groups of Examples 8 to 13 showed superior skin permeability to that of Comparative Example 1. Particularly, it was confirmed that as the proportion of malonic acid increased, skin permeability increased.

Skin permeability of the "transdermal preparation of Comparative Example 3" and the "transdermal preparation of Example 23", which were prepared via Preparation Example 1, was measured. A specific skin permeability test was performed in the same manner as in the method described in 4-1.

As a result, as shown in FIG. 9, it was confirmed that skin permeability of the transdermal preparation of Example 23 including the eutectic mixture was higher than that of the transdermal preparation of Comparative Example 3 by a factor of about at least 6, and it was confirmed that excellent skin permeability may be exhibited even when the eutectic mixture is applied to a transdermal preparation in a patch formulation.

The above description of the present invention is provided for the purpose of illustration, and it would be understood by those skilled in the art that various changes may be made without changing technical spirit and essential features of the present invention.

Therefore, it should be understood that the embodiments described above are illustrative and non-limiting in all respects. For example, each component described as a single type may be implemented in a distributed manner, and similarly, components described as being distributed may also be implemented in a combined form.

The scope of the present invention is defined by the following claims, and it shall be understood that all modifications or modified embodiments conceived from the meaning and scope of the claims and their equivalents are included in the scope of the present invention.

## Claims

1. A donepezil eutectic mixture comprising donepezil and coformer, wherein the coformer is dicarboxylic acid or vanilin.

2. The donepezil eutectic mixture of claim 1, wherein the dicarboxylic acid is at least one selected from the group consisting of adipic acid, malic acid, malonic acid, succinic acid, tartaric acid, azelaic acid, glutaric acid, itaconic acid, maleic acid, phthalic acid, pimelic acid, sebacic acid, suberic acid, and α-ketoglutaric acid.

3. The donepezil eutectic mixture of claim 1, wherein the donepezil and the coformer are included in a weight ratio of 100:1 to 1:4.

4. The donepezil eutectic mixture of claim 1, wherein the eutectic mixture is in a fluidized state at -70 °C to 250 °C.

5. A pharmaceutical composition for prevention or treatment of Alzheimer's or dementia, the pharmaceutical composition comprising the eutectic mixture of any one of claims 1 to 4.

6. The pharmaceutical composition of claim 5, wherein the pharmaceutical composition is a transdermal preparation.

7. The pharmaceutical composition of claim 6, wherein the transdermal preparation is in a form of a patch or a film.

8. A method of manufacturing a donepezil eutectic mixture, the method comprising mixing donepezil and coformer, wherein the coformer is dicarboxylic acid or vanilin.

9. The method of claim 8, wherein the donepezil and the coformer are mixed in a weight ratio of 10:1 to 1:2.
